(19) Europäisches Patentamt
European Patent Office
Office européen des brevets

(11) **EP 3 019 080 B1**

(12) **EUROPÄISCHE PATENTSCHRIFT**

(45) Veröffentlichungstag und Bekanntmachung des Hinweises auf die Patenterteilung:
**25.01.2017 Patentblatt 2017/04**

(21) Anmeldenummer: **15702706.1**

(22) Anmeldetag: **27.01.2015**

(51) Int Cl.:
*A61B 5/0476* (2006.01)   *A61B 5/00* (2006.01)

(86) Internationale Anmeldenummer:
**PCT/EP2015/051593**

(87) Internationale Veröffentlichungsnummer:
**WO 2015/121059 (20.08.2015 Gazette 2015/33)**

(54) **VERFAHREN ZUR AUTOMATISCHEN AUSWERTUNG EINES ABSENS-EEG, COMPUTERPROGRAMM UND AUSWERTEGERÄT DAFÜR**

METHOD FOR AUTOMATICALLY EVALUATING AN ABSENCE EEG, COMPUTER PROGRAM AND EVALUATING DEVICE THEREFOR

PROCÉDÉ D'ÉVALUATION AUTOMATIQUE D'UN EEG DE DIAGNOSTIC D'ABSENCES, PROGRAMME INFORMATIQUE ET APPAREIL D'ÉVALUATION CORRESPONDANT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.02.2014 DE 102014101814**

(43) Veröffentlichungstag der Anmeldung:
**18.05.2016 Patentblatt 2016/20**

(73) Patentinhaber: **Schultz, Arthur**
**29352 Adelheidsdorf (DE)**

(72) Erfinder: **Schultz, Arthur**
**29352 Adelheidsdorf (DE)**

(74) Vertreter: **Günther, Constantin**
**Gramm, Lins & Partner**
**Patent- und Rechtsanwälte PartGmbB**
**Freundallee 13 a**
**30173 Hannover (DE)**

(56) Entgegenhaltungen:
**WO-A2-97/15013**

• **A. SCHULTZ ET AL: "Age-related effects in the EEG during propofol anaesthesia", ACTA ANAESTHESIOLOGICA SCANDINAVICA, Bd. 48, Nr. 1, 12. Januar 2004 (2004-01-12), Seiten 27-34, XP055176542, ISSN: 0001-5172, DOI: 10.1111/j.1399-6576.2004.00258.x**
• **ADELAIDA LAMAS ET AL: "Assessing sedation in critically ill children by bispectral index, auditory-evoked potentials and clinical scales", INTENSIVE CARE MEDICINE, SPRINGER, BERLIN, DE, Bd. 34, Nr. 11, 4. Juli 2008 (2008-07-04), Seiten 2092-2099, XP019651657, ISSN: 1432-1238, DOI: 10.1007/S00134-008-1198-1**
• **B DE JONGHE ET AL: "Using and understanding sedation scoring systems: a systematic review", INTENSIVE CARE MEDICINE, Bd. 26, Nr. 3, 1. März 2000 (2000-03-01), Seiten 275-285, XP055176562,**

**Beschreibung**

Die Erfindung betrifft ein Verfahren zur automatischen Auswertung eines Absens-EEG gemäß dem Anspruch 1. Die Erfindung betrifft ferner ein Computerprogramm zur Durchführung eines solchen Verfahrens gemäß dem Anspruch 11 sowie ein Auswertegerät zur Auswertung eines Absens-EEG gemäß dem Anspruch 12.

Allgemein betrifft die Erfindung das Gebiet der automatischen Auswertung eines Absens-EEG, wie dies bereits anhand der WO 97/15013 A2, der WO 2010/034305 A1 oder der WO 2010/034270 A1 beschrieben ist. EEG ist dabei die Kurzform des Begriffs Elektroenzephalogramm. Hierbei wird aus EEG-Kurven, d. h. aus zeitlich nacheinander aufgenommenen Werten von EEG-Signalen eines Patienten, mittels rechnerischer Verfahren, z. B. anhand statistischer Methoden, eine Klasseneinteilung des EEG vorgenommen, bei der die aktuelle Tiefe des Absens-Zustands des Patienten bestimmt und ein aktuelles Stadium des Absens-Zustands anhand der Klasseneinteilung ermittelt und ausgegeben wird. Als Absens-Zustand wird dabei jeder Zustand des Patienten verstanden, bei dem dieser nicht oder nur eingeschränkt altersentsprechend ansprechbar/kontaktierbar ist, dies ist gegeben, wenn der Patient sich nicht im Wachzustand befindet.

Typische Absens-Zustände liegen z. B. bei einer Narkose, z. B. während einer Operation, oder bei einer sonstigen Sedierung des Patienten vor. Hierbei wird ein Patient durch Zuführung von Narkosemitteln narkotisiert bzw. sediert. Allgemein gesagt sind Narkose und Sedierung Zustände, die durch Verabreichung von schlafinduzierenden Medikamenten hervorgerufen werden. Erhalten Intensivpatienten schlafinduzierende Medikamente, dann spricht man i. A. von Sedierung. Der Begriff Sedierung ist aber nicht auf Intensivpatienten begrenzt. Wenn z. B. bei diagnostischen Eingriffen Patienten schlafinduzierende Medikamente in niedriger Dosierung erhalten, dann spricht man auch von Sedierung. Hierzu werden z. B. intravenös zu verabreichende schlafinduzierende Substanzen gegeben. Alternativ können bei Narkose und Sedierung volatile Anästhetika auf Fluranbasis, wie Sevofluran, eingesetzt werden. Diese können mit zunehmender Dosierung Krampfpotenziale auslösen, was sich in den aufgenommenen EEG-Kurven bei entsprechender Auswertung erkennen und gegebenenfalls ausblenden lässt, wie in dem zuvor genannten Stand der Technik bereits erläutert.

Weitere typische Absens-Zustände liegen z. B. bei einem Koma vor, oder bei vielen weiteren Zuständen in der Intensivpflege, d. h. auf einer Intensivstation (Intensiv-EEG). Intensivpatienten können unterschiedlichste Erkrankungen haben, die die Hirnfunktion beeinflussen können. Typischerweise kommt es im EEG mit einer Zunahme der Dämpfung der Hirnfunktion zu einer fortschreitenden Verlangsamung. Nach dem Grad der Verlangsamung kann man unterschiedliche Stadien unterscheiden, z. B. A bis F. Verlangsamungen bis hin zum Stadium F können verursacht sein durch z. B. Stoffwechselentgleisungen, Hypothermie, Sauerstoffmangel.

Allgemein umfasst der Begriff des Absens-EEG bzw. des Absens-Zustands daher Fälle, in denen die Hirnfunktion eines Patienten gegenüber einem Normalzustand im Sinne einer Dämpfung verändert ist. Neben dem oben beschriebenen Schema der Verlangsamung können im EEG Sondermuster, wie epilepsietypische Aktivität, auftreten.

Die erfassten EEG-Kurven unterliegen zudem durch eine fortschreitende Entwicklung eines Menschen gewissen Veränderungen. Besonders deutliche Veränderungen sind in der Entwicklung junger Menschen, d. h. von Kindern, bis hin zum Übergang in das Erwachsenenalter festzustellen. Insbesondere innerhalb des ersten Lebensjahres entwickeln sich die EEG-Kurven sehr deutlich.

Aus der WO 97/15013 A2 ist bereits bekannt, dass das EEG eines Menschen altersabhängige Charakteristika aufweist. Zur Verbesserung der Stadieneinteilung wurde dort vorgeschlagen, aus gespeicherten unterschiedlichen altersabhängigen Klassifikationsfunktionen die für einen Probanden altersspezifischen Klassifikationsfunktionen auszuwählen. Hierdurch kann die richtige Stadieneinteilung zuverlässiger getroffen werden.

Das bekannte Verfahren soll weiter verbessert werden, um insbesondere bei sehr jungen Patienten innerhalb der ersten Lebensjahre eine zuverlässige Bestimmung des aktuellen Stadiums des Absens-Zustands zu ermöglichen.

Diese Aufgabe wird gemäß Anspruch 1 gelöst durch ein Verfahren zur automatischen Auswertung eines Absens-EEG, bei dem EEG-Kurven von einem Auswertegerät aufgenommen und mittels eines Rechners des Auswertegerätes ausgewertet werden, wobei bei der Auswertung aus den EEG-Kurven mittels des Rechners anhand einer Stadieneinteilung des Absens-EEG wenigstens das aktuelle Stadium des Absens-Zustands eines Patienten bestimmt wird, wobei in der Stadieneinteilung Stadien der Tiefe des Absens-Zustands unterschieden werden, und wobei das aktuelle Stadium ausgegeben wird, wobei vom Rechner ein bestimmtes Schema der Stadieneinteilung aus mehreren auswählbaren Schemata von Stadieneinteilungen, die sich durch die Anzahl der unterscheidbaren Stadien des Absens-Zustands unterscheiden, ausgewählt wird und für die automatische Durchführung der Stadieneinteilung zur Bestimmung des aktuellen Stadiums verwendet wird. Die Erfindung hat den Vorteil, dass eine entwicklungs- oder altersangepasste Stadieneinteilung des Absens-EEG durchgeführt werden kann und entsprechend angepasste Informationen dem Benutzer angezeigt werden können. Diese werden mit hoher Zuverlässigkeit bestimmt. So kann insbesondere eine flexible Anpassung an sehr junge Patienten erfolgen. Bei Neugeborenen oder sehr jungen Kindern sind, wie neuere Erkenntnisse zeigen, nur wenige Absens-EEG-Stadien unterscheidbar. In Abhängigkeit von der Reife des Gehirns nimmt die Anzahl der unterscheidbaren Stadien zu.

Das Auswertegerät kann z. B. als kompaktes Gerät ausgebildet sein, das in der Nähe eines Patienten angeordnet wird. Das Auswertegerät kann auch als aus mehreren Komponenten bestehendes Gerät ausgebildet sein, wobei die Komponenten auch verteilt, z. B. in verschiedenen Räumen eines Gebäudes, angeordnet sein können. So kann z. B. das Auswertegerät eine Datenaufnahmestation zur Aufnahme der EEG-Kurven und einen entfernt davon angeordneten Rechner, z. B. einen zentralen Rechner auf einer Intensivstation für eine online-Auswertung der EEG-Kurven oder einen Rechner für eine offline-Auswertung der EEG-Kurven, aufweisen.

Gemäß einer vorteilhaften Weiterbildung der Erfindung ist vorgesehen, nach Beginn des Absens-Zustands zu prüfen, ob die EEG-Kurven bestimmte Merkmale aufweisen, anhand derer entschieden werden kann, welches Schema der Stadieneinteilung fortan genutzt wird. Die Auswahl des bestimmten Schemas aus mehreren auswählbaren Schemata von Stadieneinteilungen kann daher einmalig kurz nach Beginn des Absens-Zustands erfolgen. Es ist auch möglich, die EEG-Kurven während des Absens-Zustands weiterhin auf Merkmale zu analysieren, anhand derer das Schema der Stadieneinteilung auszuwählen ist, und im späteren Betrieb gegebenenfalls von einem einmal ausgewählten Schema noch auf ein anderes ausgewähltes Schema zu wechseln.

Das anhand des ausgewählten Schemas der Stadieneinteilung bestimmte aktuelle Stadium des Absens-Zustands wird dabei ausgegeben, z. B. indem es über eine Schnittstelle des Auswertegeräts an ein anderes Gerät übertragen wird oder direkt am Auswertegerät visuell dargestellt wird, z. B. auf einem Display. Gemäß einer vorteilhaften Weiterbildung der Erfindung wird zusätzlich eine Information über das aktuell ausgewählte Schema der Stadieneinteilung ausgegeben, z. B. durch Ausgabe an die genannte Schnittstelle oder visuelle Darstellung am Auswertegerät. Dies erlaubt dem Benutzer des Auswertegerätes eine schnelle und intuitive Einschätzung der ausgegebenen Daten.

Für die Auswertung der EEG-Kurven mittels des Rechners und die Stadieneinteilung des Absens-EEG kommen verschiedene Verfahren und Algorithmen in Frage, von denen nachfolgend beispielhaft einige erläutert werden.

Die Elektroenzephalographie ist eine Methode zur Darstellung vom Gehirn erzeugter elektrischer Aktivitäten. In konventioneller Weise erfolgt die Registrierung des EEG mit einem Mehrkanalschreiber auf Endlospapier. Zunehmend wird die Aufzeichnung auch mit Hilfe von Rechnern vorgenommen.

Die Zusammensetzung der Wellenformen im Elektroenzephalogramm (EEG) ist abhängig vom Funktionszustand des Gehirns. Die EEG-Bilder, die bei Patienten im Operations- und Intensivbereich auftreten, sind vielfältig und können durch eine große Zahl von endogenen und exogenen Faktoren beeinflusst werden. Neben dem normalen Wach-EEG ist z. B. mit Elementen des Schlaf-EEG, Effekten von Medikamenten und anderen exogen zugeführten chemischen Substanzen, ventilationsbedingten und metabolischen Einflüssen, Temperatureffekten, Folgen traumatischer Hirnläsionen sowie entzündlichen, vaskulären, degenerativen und durch die Neoplasmen verursachten EEG-Veränderungen zu rechnen.

Folgenden Frequenzbereichen werden die im EEG auftretenden Wellen zugeordnet: Alpha (7.5 - 12.5 Hz), Beta (> 12.5 Hz), Theta (3.5 - 7.5 Hz) und Delta (0.5 - 3.5 Hz). Daneben können das Subdelta- (< 0.5 Hz) und das Gamma-Band (> 30 Hz) abgegrenzt werden. Bei der Befundung werden die Wellen in Frequenzbereichen hinsichtlich ihrer Amplituden, Häufigkeit, Regelmäßigkeit, zeitlichen Gliederung, örtlichen Verteilung und Veränderung bei Reizen beschrieben. EEG-Amplituden werden in $\mu$V gemessen. Höherfrequente Wellen weisen in der Regel kleinere Amplituden auf, während mit einer Verlangsamung meist eine Amplitudenzunahme verbunden ist.

Zur Klassifikation von Schlaf-, Narkose- bzw. Koma-EEG-Stadien schlägt Kugler eine EEG-Einteilung vor, in der der Wachzustand mit A und EEG-Bilder bei fortschreitender Dämpfung der Hirnfunktion mit den Buchstaben B bis F bezeichnet werden. Zur Beurteilung der EEG-Kurven werden die Häufigkeit und Amplitude der Wellen in bestimmten Frequenzbereichen sowie typische Muster herangezogen.

Das Wach-EEG, Stadium A, ist bei der Mehrzahl der Erwachsenen durch Wellen im Alpha-Frequenzbereich gekennzeichnet. Stadium B ist charakterisiert durch Wellen mit schneller Frequenz und niedriger Amplitude. In den Stadien C bzw. D treten Theta- und Delta-Wellen auf. Im Stadium E bestimmt hochamplitudige Delta-Aktivität das Kurvenbild. Das Stadium F ist durch einen Wechsel flacher bis isoelektrischer Kurvenstrecken und Gruppen höherer Wellen, das Burst-Suppression-Muster, oder durch eine kontinuierliche sehr flache Aktivität geprägt.

Die Ableitung eines konventionellen EEG ist relativ aufwändig. Die Interpretation erfordert spezielle Kenntnisse und Erfahrung. Eine bessere Beurteilung der dynamisch ablaufenden EEG-Veränderung wird durch die Aufzeichnung des Original-Signals und der EEG-Spektralanalyse ermöglicht. Für eine Berechnung eines EEG-Leistungsspektrums werden für einen definierten Zeitabschnitt die EEG-Signale nach Analog-Digital-Wandlung z. B. einer Fast Fourier-Transformation (FFT) unterzogen. Mit Hilfe der Fourier-Transformation wird das Wellenbild des EEG in zugrundeliegende Schwingungskomponenten zerlegt, es erfolgt eine Umsetzung vom Zeit- in den Frequenzbereich. Die quadrierten Amplituden der Schwingungskomponenten bilden das Leistungs- oder Powerspektrum. Im EEG-Leistungsspektrum sind die im Zeitsignal auftretenden Frequenzen ablesbar. Aber auch diese Angaben bedürfen der Interpretation, um Aufschluss über das EEG-Stadium und damit über den cerebralen Funktionszustand zu erhalten.

Die Weiterverarbeitung der Ergebnisse der Fourier-Transformation umfasst die Extraktion sogenannter Spektralparameter sowie weiterführende statistische Berechnungen. Zu den Parametern, die sich aus dem Spektrum ableiten lassen, gehören z. B. die Gesamtleistung sowie absolute und relative Leistungen in unterschiedlichen Frequenzbändern. Weitere häufig verwendete Parameter sind der Median, die Spectral Edge Frequency und die dominante Frequenz. Der Median ist die Frequenz, bei der die Fläche des Spektrums in zwei gleiche Teile geteilt wird. Die Spectral Edge Frequency wird meistens als 95 %-Quantil definiert, d. h. 95 % der Gesamtleistung des Spektrums liegen unterhalb dieser Frequenz. Die dominante Frequenz ist die Frequenz mit der größten Leistung.

Mit dem Leistungsspektrum lässt sich die Frequenzverteilung von EEG-Abschnitten übersichtlich darstellen. Auf spezielle Muster, wie Burst-Suppression-Phasen oder Anfallspotenziale, lässt sich aus dem Spektrum dagegen meist nicht schließen.

Ein Verfahren zur schnellen Berechnung des Leistungsspektrums ist die Fast Fourier-Transformation (FFT).

Eine Möglichkeit zur Analyse von EEG-Signalen im Zeitbereich ist die Ermittlung autoregressiver Parameter. Autoregressive (AR)-Parameter sind Größen aus dem Zeitbereich. Ein Messwert zu einem bestimmten Zeitpunkt wird dargestellt als gewichtete Summe seiner Vergangenheitswerte plus einer Zufallskomponente. Die Gewichte sind die AR-Parameter. Die allgemeine Formel für einen AR-Prozess lautet:

$$Y_t = a_1 * Y_{t-1} + \ldots + a_p * Y_{t-p} + e_t.$$

Hierbei bezeichnen Yt den Messwert zum Zeitpunkt t, die $a_i$, i = 1, ..., p die AR-Parameter und $e_t$ unabhängige Zufallskomponenten mit Mittelwert 0 und konstanter Varianz für alle Zeitpunkte t. Der Buchstabe p bezeichnet die Ordnung des Prozesses, d. h. die Anzahl der Vergangenheitswerte, die berücksichtigt werden. Die Modellparameter können mit Hilfe der Yule-Walker-Gleichung geschätzt werden. Zur Ermittlung der Ordnung des Modells und der Überprüfung der Modellgüte wird üblicherweise der Ansatz von Box und Jenkins verwendet. Einen Überblick über weitere Schätzverfahren und Modellklassen geben Kay und Marple.

Eine häufig eingesetzte Methode zur Charakterisierung von EEG-Messungen ist die Berechnung spezieller EEG-Parameter, die von Hjorth vorgeschlagen und nach ihm benannt worden sind. Es handelt sich dabei um drei Parameter, und zwar Aktivität, Mobilität und Komplexität. Die Hjorth-Parameter werden aus der Streuung des EEG-Signals sowie deren erster und zweiter Ableitung berechnet. Alternativ kann die Berechnung der Hjorth-Parameter auch im Frequenzbereich, d. h. mit Hilfe der Spektralanalyse vorgenommen werden.

Die Aktivität entspricht der Gesamtleistung des Signals und ist damit ein Maß für die Amplitudengröße der EEG-Messung. Die Mobilität kann interpretiert werden als ein Maß für die mittlere Frequenz und die Komplexität als ein Maß für die Variabilität des Signals.

Neben reinen Spektralparametern oder reinen AR-Parametern ist die kombinierte Ermittlung z. B. von Spektralparametern, AR-Parametern, Hjorth-Parametern oder auch Chaosparametern und/oder auch weiteren Parametern möglich.

Für die Klassifikation von EEG-Daten mittels multivariater Klassifikationsfunktionen auf der Grundlage von Spektralparametern und/oder AR-Parametern und/oder Hjorth-Parametern und/oder Chaosparametern und/oder auch weiteren Parametern eignen sich z. B. diskriminanzanalytische Verfahren oder neuronale Netze.

Diskriminanzanalytische Klassifikationsverfahren sind dazu geeignet, Objekte anhand einer Reihe erhobener Merkmale einer von mehreren definierten Gruppen zuzuordnen. Bei der Absens-EEG-Stadieneinteilung bilden die EEG-Abschnitte die zu klassifizierenden Objekte, die durch Spektralparameter und/oder AR-Paramter und/oder Hjorth-Parameter und/oder Chaosparameter charakterisiert sind. Zur Berechnung geeigneter Klassifikationsfunktionen existiert eine Reihe von Methoden, bei denen sich parametrische und nichtparametrische Ansätze unterscheiden lassen. Mittels einer Strichprobe von Objekten, für die die Gruppenzugehörigkeit bekannt ist, lassen sich Klassifikationsfunktionen basierend auf den betrachteten Merkmalswerten herleiten.

Bei parametrischen Verfahren wird vorausgesetzt, dass der betrachtete Merkmalsvektor in den verschiedenen Gruppen einer multivariaten Normalverteilung folgt. Die lineare Diskriminanzanalyse setzt die Gleichheit der Kovarianzmatrizen in den einzelnen Gruppen voraus, die quadratische Diskriminanzanalyse ermöglicht die Berücksichtigung unterschiedlicher Kovarianzmatrizen der Gruppen. Als Abstandsmaß wird die Mahalanobis-Distanz verwendet, die den gewichteten Abstand eines Beobachtungsvektors zu den Gruppenmittelwerten darstellt. Ein Objekt wird dann derjenigen Gruppe zugeordnet, bei der eine von dem gewählten Verfahren abhängige Funktion der Mahalanobis-Distanz am kleinsten ist.

Wenn die Verteilung des Merkmalsvektors unbekannt bzw. nicht normalverteilt ist, können nichtparametrische Verfahren zur Herleitung von Klassifikationsregeln eingesetzt werden. Ein anschauliches Verfahren ist die k-nearestneighbor-Methode. Hierbei werden die Abstände des zu klassifizierenden Merkmalsvektors zu allen anderen Merkmalsvektoren der zur Verfügung stehenden Stichprobe gebildet, der Größe nach geordnet und die Beobachtungsvektoren mit den k kleinsten Abständen bestimmt, wobei die Anzahl k der berücksichtigten Werte vorher festgelegt werden muss. Dann wird bestimmt, zu welchen Gruppen diese k Werte gehören und deren Anteil an der Gesamtzahl der Messungen in den einzelnen Gruppen ermittelt. Die Zuordnung erfolgt dann zu der Gruppe, bei der dieser Anteil am Größten ist.

Dieses nichtparametrische Verfahren erfordert einen erhöhten Rechenaufwand gegenüber parametrischen Methoden, da zur Klassifikation eines Objektes auf den gesamten Originaldatensatz zurückgegriffen werden muss, während bei parametrischen Methoden die Merkmalswerte eines Objektes in Klassifikationsfunktionen eingesetzt werden.

Zur Beurteilung der Güte eines Klassifikationsverfahrens kann die zugehörige Fehlerrate herangezogen werden, wobei unter Fehlerrate der Anteil von Fehlklassifikationen verstanden wird. Eine Möglichkeit zur Schätzung der Fehlerrate besteht in der Reklassifikation der Daten. Die so ermittelte Fehlerrate liefert jedoch eine zu positive Schätzung der wahren Fehlerrate. Eine realistischere Schätzung der Fehlerrate ist dann gegeben, wenn die Klassifikationen an einem unabhängigen Datensatz überprüft werden. Dies kann durch eine Aufsplittung des gegebenen Datensatzes in einen Trainingsdatensatz zur Herleitung der Klassifikationsvorschrift und einen Testdatensatz zur Validierung der Klassifikation erfolgen. Eine extreme Form des Aufsplittens der Daten besteht in der sogenannten Crossvalidation oder dem Leave-One-Out-Verfahren. Hierbei wird jeweils eine Beobachtung aus dem Datensatz herausgenommen und die Klassifikation anhand der aus den verbleibenden Daten berechneten Diskriminanzfunktion vorgenommen.

Liegt eine große Anzahl potenzieller Merkmale zur Herleitung von Diskriminanzfunktionen vor, so lassen sich mit Hilfe geeigneter stufenweiser Verfahren diejenigen Parameter ermitteln, die eine größtmögliche Trennung der Gruppen gewährleisten. Zu diesem Zweck wird in der Literatur eine Reihe von Verfahren vorgeschlagen, z. B. werden schrittweise Parameter in die Auswertung aufgenommen, die anhand von Wilks Lambda jeweils den größten Beitrag zur Gruppentrennung liefern.

Die Stadienteilung des Narkose- oder Intensiv-EEG kann in Anlehnung an Kugler erfolgen, der, wie einleitend erwähnt wurde, den Wachzustand mit A und die sehr tiefe Dämpfung der Hirnfunktion mit F bezeichnet. Die Zwischenstadien B bis E können dabei noch weiter unterteilt werden, wie die Tabelle 1 in WO 97/15013 A2 zeigt. Es ist auch möglich, statt der Klassenbezeichnungen A bis F z. B. eine Skala mit Ziffern zu verwenden, z. B. 100 bis 0.

Eine weitere Verbesserung der Stadieneinteilung wird erreicht, wenn aus gespeicherten unterschiedlichen altersabhängigen Klassifikationsfunktionen die für einen Probanden altersspezifischen Klassifikationsfunktionen ausgewählt werden. Es wurde herausgefunden, dass das EEG eines Menschen altersabhängige Charakteristika aufweist. Vereinfacht ausgedrückt verschiebt sich z. B. das Spektrum bei Erwachsenen mit zunehmendem Alter im Wachzustand zu niedrigeren Frequenzen, während der Narkose ist z. B. die Delta-Leistung vermindert. Durch Berücksichtigung altersspezifischer Klassifikationsfunktionen kann die richtige Stadieneinteilung zuverlässig getroffen werden.

Gemäß einer vorteilhaften Weiterbildung der Erfindung wird eine Angabe über das Alter des Patienten, dessen EEG-Kurven aufgenommen werden, in das Auswertegerät eingegeben. Die automatische Auswahl des Schemas der Stadieneinteilung wird vom Rechner unter Berücksichtigung der eingegebenen Altersangabe durchgeführt. Dies hat den Vorteil, dass durch die manuelle Eingabe des Alters des Patienten der Bereich der wahrscheinlich in Frage kommenden auszuwählenden Schemata von Stadieneinteilungen eingegrenzt werden kann. Allerdings hat sich gezeigt, dass eine eindeutige Festlegung des auszuwählenden Schemas von Stadieneinteilungen allein aufgrund der Altersangabe meistens nicht zu zufriedenstellenden Ergebnissen führt, da sich Menschen und deren Gehirne altersabhängig unterschiedlich entwickeln und daher keine bestimmte Altersgrenze definiert werden kann, bei der in den EEG-Kurven z. B. die bei Erwachsenen übliche Unterteilung in die Stadien A bis F vorgenommen werden kann. Untersuchungen zeigen, dass eine solche Stadieneinteilung etwa ab einem Alter von einem halben Jahr möglich ist, allerdings ist die Altersstreuung relativ groß. Bei sehr jungen Kindern ist es daher vorteilhaft, eine weniger differenzierte Stadieneinteilung zugrunde zu legen.

Gemäß einer vorteilhaften Weiterbildung der Erfindung wird die automatische Auswahl des Schemas der Stadieneinteilung vom Rechner unter Berücksichtigung der aufgenommenen EEG-Kurven und/oder daraus abgeleiteten Daten durchgeführt. Dies hat den Vorteil, dass eine zuverlässige Auswahl eines passenden Schemas der Stadieneinteilung automatisch erfolgen kann, nämlich anhand der ohnehin aufgenommenen Kurven, sodass sich die Benutzung des Auswertegerätes weiter vereinfacht. Die Berücksichtigung der aufgenommenen EEG-Kurven kann z. B. dahingehend erfolgen, dass die EEG-Kurven auf bestimmte charakteristische Kurvenmuster oder bestimmte statistische Daten, die aus den Kurven ermittelt werden können und die für bestimmte Entwicklungsstände von EEGs charakteristisch sind, untersucht werden. So kann die Auswahl des Schemas der Stadieneinteilung z. B. anhand von Amplitudendaten, Frequenzdaten und/oder Mittelwerten oder zeitlichen Verläufen von Amplituden und/oder Frequenzen der EEG-Kurven durchgeführt werden.

Gemäß einer vorteilhaften Weiterbildung der Erfindung wird bei Erkennung eines bestimmten Mindestanteils niederfrequenter Signalanteile und Nichterkennung eines bestimmten Mindestanteils hochfrequenter Signalanteile in den EEG-Kurven ein Schema mit geringerer Anzahl der unterscheidbaren Stadien des Absens-Zustands ausgewählt als bei Erkennung eines Mindestanteils hochfrequenter Signalanteile, die mit einem bestimmten Mindestanteil niederfrequenter Signalanteile kombiniert sein können. Auf diese Weise kann z. B. bei Patienten im Kindesalter, die ein überwiegend langsames Wellenbild mit entsprechend niederfrequenten Signalanteilen im EEG zeigen, ein passendes Schema der Stadieneinteilung mit weniger unterscheidbaren Stadien ausgewählt werden. Bei älteren Patienten, die durch mehr hochfrequente Signalanteile in den EEG-Kurven charakterisiert sind, können Schemata mit angepassten, feiner differenzierten Stadieneinteilungen ausgewählt werden.

Unter dem Einfluss von Narkotika/Sedativa können als niederfrequente Signalanteile Delta-Wellen auftreten. Sind diese niederfrequenten Wellen mit einem Mindestanteil von höherfrequenten Wellen - als Ausdruck der Wirkung von Narkosemitteln - überlagert, kann die Entscheidung gefällt werden, dass es sich um ein differenziertes EEG handelt. Dementsprechend kann dann ein Schema mit größerer Anzahl der unterscheidbaren Stadien des Absens-Zustands ausgewählt. Besteht das EEG lediglich aus niederfrequenten Wellen, dann sollte ein Schema mit geringerer Anzahl der unterscheidbaren Stadien ausgewählt werden.

Tritt bei jungen Kindern im Messverlauf höherfrequente Aktivität mit geringem oder nicht vorhandenem Anteil niederfrequenter Signalanteile auf, wie sie z. B. in den Stadien B und C bei älteren Kindern oder Erwachsenen typisch ist, dann wird ein Schema mit größerer Anzahl der unterscheidbaren Stadien des Absens-Zustands ausgewählt.

Gemäß einer vorteilhaften Weiterbildung der Erfindung kann bei Erkennung eines bestimmten Anteils hochfrequenter Signalanteile in den EEG-Kurven ein Schema mit größerer Anzahl der unterscheidbaren Stadien des Absens-Zustands ausgewählt werden. Allerdings sollte in dem Fall, dass "Nichterkennung eines Mindestanteils niederfrequenter Signalanteile" zutrifft und "Erkennung eines bestimmten Anteils hochfrequenter Signalanteile" nicht zutrifft, nicht ein Schema mit größerer Anzahl der unterscheidbaren Stadien des Absens-Zustands ausgewählt werden, denn es könnte sich um ein nahezu vollständig oder vollständig supprimiertes EEG ("Stadium F" mit Nulllinien) handeln.

Gemäß einer vorteilhaften Weiterbildung der Erfindung geht der Rechner zunächst von einem ersten Schema mit einer bestimmten Anzahl der unterscheidbaren Stadien des Absens-Zustands aus und wählt zu einem Beurteilungszeitpunkt, zu dem eine ausreichende Anzahl von EEG-Daten vorliegt, bei Erkennung bestimmter Charakteristika in den EEG-Kurven ein zweites Schema der Stadieneinteilung aus, das eine geringere oder größere Anzahl der unterscheidbaren Stadien des Absens-Zustands aufweist als das erste Schema. So kann z. B. bei Erkennung eines Mindestanteils von niederfrequenten Signalanteilen in den EEG-Kurven das zweite Schema der Stadieneinteilung ausgewählt werden. Tritt die Auswahlbedingung, d. h. die Erkennung bestimmter Charakteristika, nicht ein, so kann der Rechner z. B. weiterhin das erste Schema anwenden oder das erste Schema als für die automatische Durchführung der Stadieneinteilung zu verwendendes Schema auswählen. So kann als erstes Schema z. B. das Schema einer Stadieneinteilung für Erwachsene verwendet werden, z. B. mit der Stadieneinteilung A bis F oder die feinere Einteilung mit den Stadien $A_0$, $A_1$, $A_2$, $B_0$, $B_1$, $B_2$, $C_0$, $C_1$, $C_2$, $D_0$, $D_1$, $D_2$, E und F, wie in Tabelle 1 von WO 97/15013 A2 beschreiben. Das zweite Schema der Stadieneinteilung kann z. B. eine Einteilung in die Stadien A, E und F haben. Die zuvor genannte Zuordnung der Stadieneinteilungen zu dem ersten und dem zweiten Schema kann auch umgekehrt erfolgen.

Für die Festlegung des ersten Schemas, von dem der Rechner zunächst ausgeht, kann auch die eingegebene Altersangabe des Patienten verwendet werden. Bei sehr kleinen Kindern, insbesondere bei Neu- bzw. Frühgeborenen, ist davon auszugehen, dass zunächst eine Stadieneinteilung mit reduzierter Stadienanzahl verwendbar ist. Das dürfte bei allen Kindern in den ersten zwei bis drei Lebensmonaten der Fall sein, wie aktuelle eigene EEG-Analysen zeigen. Bei entsprechender Alterseingabe geht das Auswertegerät nicht von der Standardeinteilung A, B, ..., F bzw. 100 bis 0 aus, sondern von einem Schema mit reduzierter Stadieneinteilung. Bei älteren Kindern kann das Auswertegerät zunächst von einem Schema mit größerer Anzahl der unterscheidbaren Stadien ausgehen und dann während der Messung ggf. auf ein Schema mit geringerer Anzahl der unterscheidbaren Stadien übergehen.

Gemäß einer vorteilhaften Weiterbildung der Erfindung werden wenigstens Frequenzanteile im Delta-Band als niederfrequente Signalanteile der EEG-Kurven gewertet. Zusätzlich können auch Frequenzanteile mit Frequenzen unterhalb des Delta-Bands als niederfrequente Signalanteile gewertet werden. In einer Weiterbildung der Erfindung können Frequenzanteile mit Frequenzen oberhalb des Delta-Bands als hochfrequente Signalanteile gewertet werden.

Als weiteres Kriterium für die automatische Auswahl eines Schemas der Stadieneinteilung können die EEG-Kurven im Hinblick auf sogenannte Suppressionsstrecken in Burst-Suppression-Mustern oder im Suppressions-EEG ausgewertet werden. Als Burst wird dabei eine Folge von Signalwellen in einer EEG-Kurve verstanden. Suppressionsstrecken in EEG-Kurven sind Kurvenabschnitte, bei denen keine Bursts auftauchen und das Signal einen im Vergleich zu den Signalwellen eines Bursts flachen Verlauf hat. Die Abschnitte zwischen benachbarten Bursts werden als Suppressionsstrecken bezeichnet. Gemäß einer vorteilhaften Weiterbildung der Erfindung kann bei der Auswertung der EEG-Kurven eine Erkennung von Burst-Suppression-Mustern durchgeführt werden und bei Auftreten vorbestimmter Charakteristika von Burst-Suppression-Mustern in den EEG-Kurven ein Schema der Stadieneinteilung ausgewählt werden, das eine verringerte Anzahl der unterscheidbaren Stadien in des Absens-Zustands aufweist, im Vergleich zu einem sonst ausgewählten Schema der Stadieneinteilung. Als Charakteristika von Burst-Suppression-Mustern kann z. B. die Länge von Suppressionsstrecken oder die Burst-Suppression-Ratio (BSR) herangezogen werden. Die Burst-Suppression-Ratio gibt an, wie viel Prozent eines EEG-Kurvenabschnitts aus Suppressionsstrecken besteht. Ferner kann das Inter-Burst-Intervall (IBI), das ein Maß für den Abstand zwischen Bursts ist, als Charakteristikum herangezogen werden.

Gemäß einer vorteilhaften Weiterbildung der Erfindung sind außer einem Standardschema der Stadieneinteilung ein weiteres Schema oder je nach Entwicklungsstufe des Patienten mehrere weitere Schemata der Stadieneinteilung vom Rechner auswählbar, insbesondere Stadieneinteilungen mit einer geringeren Anzahl an unterscheidbaren Stadien des Absens-Zustands als das Standardschema. Dies erlaubt eine besonders gut an den Entwicklungsstand des EEG angepasste Stadieneinteilung.

Vorteilhaft ist insbesondere die Aufnahme von EEG-Daten bis zu dem Beurteilungspunkt nach der Einleitung des Absens-Zustands. Hierdurch werden mögliche Verfälschungen bei der Auswahl eines zutreffenden Schemas der Stadieneinteilung durch noch im Wachzustand aufgenommene EEG-Daten vermieden. Untersuchungen zeigen, dass es praktisch nicht möglich ist, anhand des Wach-EEG eine entwicklungsbezogene Stadieneinteilung eines Absens-EEG auszuwerten.

Gemäß einer vorteilhaften Weiterbildung der Erfindung wird anhand des ausgewählten Schemas der Stadieneinteilung eine Angabe über den Entwicklungsstand des EEG bestimmt. Diese Angabe über den Entwicklungsstand des EEG kann im Auswertegerät intern weiterverwendet werden, um hiervon abhängig bestimmte weitere Auswertungen oder Klassifikationsfunktionen der Analyse der EEG-Signale zu beeinflussen. Die Angabe über den Entwicklungsstand des EEG kann auch ausgegeben werden, z. B. über eine Schnittstelle des Auswertegeräts an ein anderes Gerät oder visuell dargestellt werden, z. B. auf einem Display des Auswertegeräts.

Gemäß einer vorteilhaften Weiterbildung der Erfindung werden die EEG-Kurven auf durch weitere Biosignale erzeugte Kurvenmuster analysiert und, wenn wenigstens ein solches Kurvenmuster erkannt wird, wird geprüft, ob ein anderes Schema für die Stadieneinteilung auszuwählen ist als bei Nichterkennung solcher Kurvenmuster. Auf diese Weise können z. B. Signaleinstreuungen von außen, Artefakte, insbesondere Bewegungsartefakte sowie epilepsietypische Potenziale detektiert werden und sowohl für die Auswahl des Schemas der Stadieneinteilung als auch für die eigentliche Stadienklassifikation, d. h. die Klassifikationsfunktion, berücksichtigt werden.

Gemäß einer vorteilhaften Weiterbildung der Erfindung werden mittels Artefaktsensoren, die an das Auswertegerät angeschlossen sein können, Bewegungsartefakte in den aufgenommenen EEG-Kurven ermittelt und anhand der ermittelten Bewegungsartefakte die EEG-Kurven korrigiert und/oder die Stadieneinteilung korrigiert und/oder unterdrückt und/oder ein anderes Schema der Stadieneinteilung ausgewählt. Solche Artefaktsensoren können z. B. als Verformungssensoren von EEG-Elektroden ausgebildet sein. Solche Verformungssensoren können Kapazitäten umfassen, die durch Verformung veränderbar sind und deren Kapazitätsänderung mit der Verformung der EEG-Elektroden korreliert.

Für die Bestimmung des aktuellen Stadiums des Absens-Zustands und/oder für die Auswahl des zu verwendenden Schemas der Stadieneinteilung können die EEG-Kurven entweder direkt verwendet werden oder daraus abgeleitete Daten. So können z. B. Frequenzanteile durch eine Fourier-Analyse oder eine ähnliche Analyse bestimmt werden. Es können Amplitudenwerte statistisch ausgewertet werden. Es kann auch ein Amplituden-integriertes EEG bestimmt werden. Das Amplituden-integrierte EEG ist eine zeitkomprimierte Darstellung von Amplituden eines EEG-Abschnitts. Bei der Berechnung des Amplituden-integrierten EEG kann das EEG-Signal z. B. stark gefiltert, gleichgerichtet und geglättet werden.

Die eingangs genannte Aufgabe wird gemäß Anspruch 11 ferner gelöst durch ein Computerprogramm mit Programmcodemitteln, eingerichtet zur Durchführung eines Verfahrens der zuvor beschriebenen Art, wenn das Computerprogramm auf einem Rechner ausgeführt wird. Das Computerprogramm kann insbesondere auf einem Rechner des zuvor erläuterten Auswertegerätes ausgeführt werden. Das Computerprogramm kann dabei auf einem maschinenlesbaren Träger gespeichert sein, z. B. auf einer CD oder DVD, einem Speicherstick, auf einem Internet-Server oder auf einem Speichermedium des Auswertegerätes.

Die eingangs genannte Aufgabe wird gemäß Anspruch 12 ferner gelöst durch ein Auswertegerät zur Auswertung eines Absens-EEG, wobei das Auswertegerät wenigstens einen Rechner, EEG-Signalerfassungsmittel und Ausgabemittel aufweist, wobei das Auswertegerät zur Durchführung eines Verfahrens der zuvor beschriebenen Art eingerichtet ist. So kann das Auswertegerät z. B. zur Ausführung des Verfahrens eingerichtet sein, indem der Rechner ein Computerprogramm der zuvor beschriebenen Art ausführt. Das Ausgabemittel kann z. B. eine Schnittstelle des Auswertegeräts oder ein Mittel zur visuellen Darstellung, z. B. ein Display sein.

Die Erfindung wird nachfolgend anhand von Ausführungsbeispielen unter Verwendung von Zeichnungen näher erläutert.

Es zeigen:

Figur 1    ein Auswertegerät bei der Aufnahme eines EEG und

Figur 2    eine Darstellung von durch das Auswertegerät aufgenommenen EEG-Kurven und daraus abgeleiteten Daten und

Figur 3    eine Darstellung des Ablaufs bei der Auswertung eines Absens-EEG in dem Auswertegerät.

In den Figuren werden gleiche Bezugszeichen für einander entsprechende Elemente verwendet.

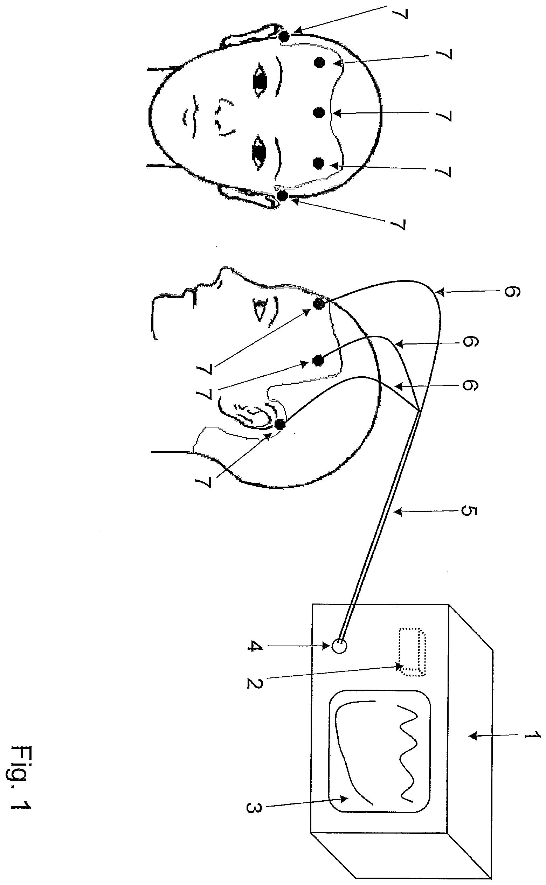

Die Figur 1 zeigt ein Auswertegerät 1 zur Auswertung eines Absens-EEG. Das Auswertegerät 1 weist einen im Auswertegerät z. B. auf einer zentralen Platine angeordneten Rechner 2 auf, der z. B. als Mikroprozessor oder Mikrocontroller ausgebildet sein kann. Das Auswertegerät 1 weist ferner ein Anzeigemittel 3 auf, z. B. ein Display. Auf dem Anzeigemittel 3 können Kurvenverläufe graphisch dargestellt werden oder sonstige erfasste und daraus bestimmte Daten wiedergegeben werden. Das Auswertegerät 1 weist ferner einen elektrischen Anschluss 4 auf, der zum Anschließen von EEG-Elektroden 7 dient, z. B. über Steckverbinder.

In Figur 1 ist dargestellt, wie mehrere EEG-Elektroden 7 am Kopf eines Patienten angeordnet sind. Die EEG-Elektroden 7 sind über Kabel 6, die in der Nähe des Auswertegeräts zu einem gemeinsamen Kabelstrang 5 vereinigt werden, mit dem elektrischen Anschluss 4 des Auswertegeräts 1 verbunden. Der gemeinsame Kabelstrang 5 kann z. B. durch Auswertung der zwischen den Kabeln vorhandenen Kapazitätswerte als einer oder mehrere Artefaktsensoren zur Erkennung von Bewegungsartefakten verwendet werden. Hierzu sind im Auswertegerät 1 Erfassungsmittel zur Erfassung von Kapazitäten zwischen den Leitungen 6 des Kabelstrangs 5 vorgesehen.

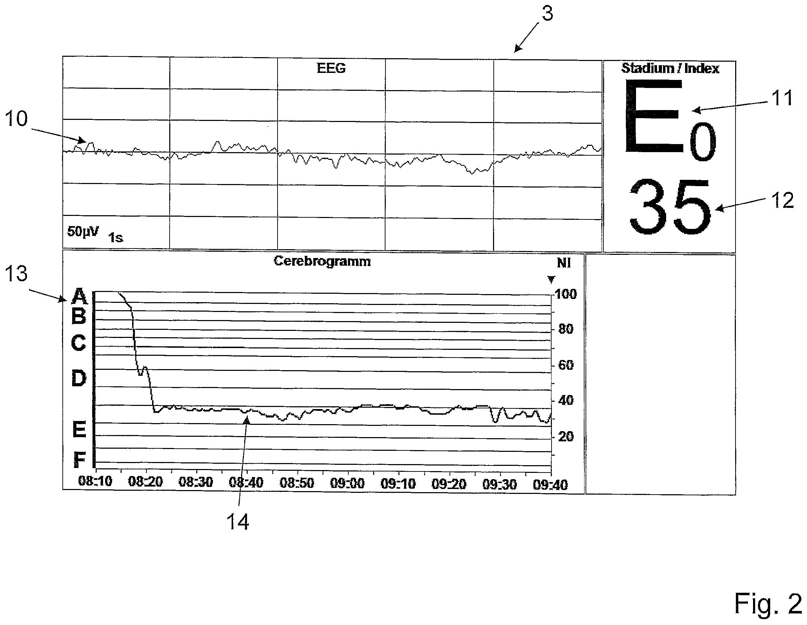

Die Figur 2 zeigt beispielhaft typische auf dem Anzeigemittel 3 des Auswertegeräts 1 ausgegebene Daten. In einem oberen Fenster können z. B. eine oder mehrere EEG-Kurven 10, so wie sie von den EEG-Sensoren 7 aufgenommen werden, als Kurvenverlauf über die Zeit dargestellt werden. In einem Anzeigebereich 11 kann das aktuelle Stadium des Absens-Zustands, wie es vom Rechner 2 durch Auswertung der EEG-Kurven ermittelt wird, dargestellt werden. In einem Anzeigebereich 12 kann die Narkose- bzw. Sedierungstiefe zusätzlich als dimensionslose Zahl im Bereich von 0 bis 100 ausgegeben werden. In einer Zeitdiagrammdarstellung rechts unten kann der zeitliche Verlauf der ermittelten Stadien, wie in Bereich 11 dargestellt, als Kurvenverlauf 14 angegeben werden. An der Vertikalachse des Diagramms sind im Bereich 13 die unterscheidbaren Stadien A bis F des ausgewählten Schemas der Stadieneinteilung wiedergegeben. Die Angabe A bis F deutet auf die Stadieneinteilung bei einem erwachsenen Patienten hin. Bei einem weniger entwickelten EEG, z. B. bei einem sehr jungen Kind, kann im Bereich 13 dann aufgrund eines vom Rechner ausgewählten anderen Schemas der Stadieneinteilung eine andere Ausgabe erfolgen, z. B. nur die Buchstaben A, E und F. Es ist auch möglich, statt der Klassenbezeichnungen A bis F z. B. eine Skala mit Ziffern zu verwenden, z. B. 100 bis 0.

Die nachfolgende Tabelle zeigt eine beispielhafte Zuordnung zwischen den Stadien mit den Klassenbezeichnungen A bis F und einer Skala mit Ziffern (Index-Werte).

| Stadium | Index | Dominierende EEG-Charakteristika |
|---------|-------|----------------------------------|
| A | 100 - 95 | Alpha-Wellen |
| $B_0$ | 94 - 90 | Beta-Wellen, Theta-Wellen |
| $B_1$ | 89 - 85 | |
| $B_2$ | 84 - 80 | |
| $C_0$ | 79 - 75 | Zunehmende Menge von Theta-Wellen |
| $C_1$ | 74 - 70 | |
| $C_2$ | 69 - 65 | |
| $D_0$ | 64 - 57 | Zunehmende Menge von Delta-Wellen |
| $D_1$ | 56 - 47 | |
| $D_2$ | 46 - 37 | |
| $E_0$ | 36 - 27 | Kontinuierliche hohe Delta-Wellen |
| $E_1$ | 26 - 20 | |
| $E_2$ | 19 - 13 | Übergang zum Burst-Suppression-Muster |
| $F_0$ | 12 - 5 | Burst-Suppression-Muster |
| $F_1$ | 4 - 0 | Kontinuierliche EEG-Suppression |

Wie schon erläutert, sollte bei Kindern mit unreifem EEG eine Stadieneinteilung mit wenigen Stadien verwendet werden (hier beispielhaft A, E, F). Die Indexwerte 100 - 0 könnten entweder an die reduzierte Stadieneinteilung angepasst werden (der gesamte Indexbereich 100 - 0 würde genutzt), oder es könnte nur ein Teil des Indexbereiches 100 - 0 genutzt werden, z. B. nur der Bereich 100 - 95 und der Bereich 36 - 0 würde genutzt.

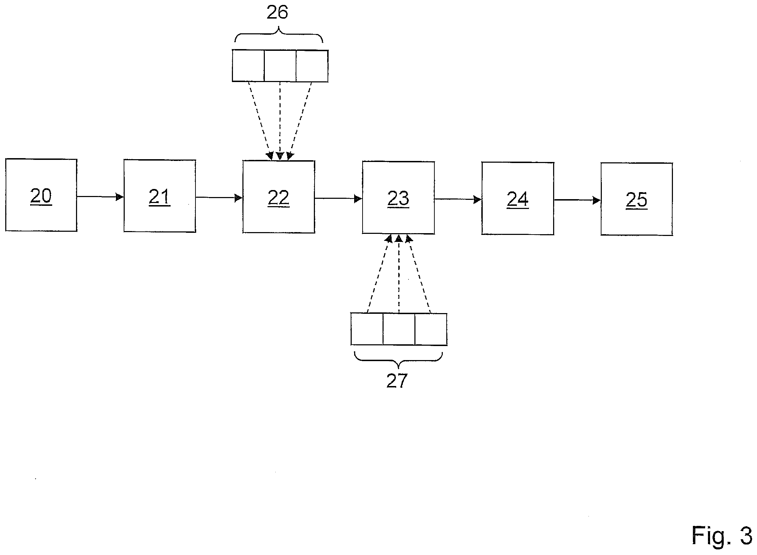

Die Figur 3 zeigt den Ablauf bei der automatischen Auswertung der EEG-Kurven durch den Rechner 2. Die in Figur 3 wiedergegebenen Blöcke 20, 21, 22, 23, 24, 25 geben dabei bestimmte Auswertefunktionen oder Algorithmen an, die auf dem Rechner 2 ausgeführt werden. So können die Blöcke 20, 21, 22, 23, 24, 25 z. B. als Programmabschnitte, Programmmodule oder Unterprogramme eines Computerprogramms, das der Rechner 2 ausführt, ausgebildet sein.

Im Block 20 erfolgt ein Einlesen der EEG-Kurven. In einem darauf folgenden Block 21 erfolgt eine Signalanalyse der EEG-Kurven, z. B. durch Fourier-Transformation, Bestimmung eines Leistungsspektrums und/oder eines Amplituden-integrierten EEG. In einem darauf folgenden Block 22 erfolgt anhand eines oder mehrerer der nachfolgend noch erläuterten Kriterien eine Auswahl eines anzuwendenden Schemas der Stadieneinteilung aus mehreren auswählbaren Schemata 26. Beispielhaft sind durch die drei Blöcke 26 drei auswählbare Schemata dargestellt, von denen der Rechner 2 im Block 22 eines auswählt.

In einem darauf folgenden Block 23 erfolgt eine Auswahl einer Klassifikationsfunktion für die nachfolgend durchzuführende Klassifikation der EEG-Kurven und zur Ermittlung des aktuellen Stadiums anhand einer Stadieneinteilung des Absens-EEG. Beispielhaft ist dargestellt, dass der Rechner im Block 23 eine von drei auswählbaren Klassifikationsfunktionen 27 auswählen kann. In einem darauf folgenden Block 24 erfolgt dann eine Auswertung der EEG-Kurven bzw. daraus bestimmter Daten anhand der ausgewählten Klassifikationsfunktion derart, dass eine Stadieneinteilung des Absens-EEG unter Anwendung des in Block 22 ausgewählten Schemas der Stadieneinteilung durchgeführt wird. In einem darauf folgenden Block 25 wird dann, gegebenenfalls neben anderen Daten, das ermittelte aktuelle Stadium des Absens-Zustands ausgegeben.

Im Block 22 kann die Auswahl des zu verwendenden Schemas der Stadieneinteilung aus den verfügbaren Schemata 26 anhand eines vorab eingegebenen Alters des Patienten, dessen EEG-Kurven ausgenommen werden, anhand der aufgenommenen EEG-Kurven und/oder daraus abgeleiteten Daten selbst und/oder anhand von zuvor ausgewählten Schemata von Stadieneinteilungen durchgeführt werden, wie zuvor bereits im Detail näher erläutert. Insbesondere können hierzu EEG-Kurven nach Einleitung des Absens-Zustands verwendet werden. Auf diese Weise kann eine an die Entwicklung des EEG bzw. die Entwicklung des Patienten und dessen Alters angepasste Stadieneinteilung erfolgen. Analog dazu kann im Block 23 eine entsprechend angepasste Auswahl einer Klassifikationsfunktion aus den verfügbaren Klassifikationsfunktionen 27 erfolgen. Die Kriterien bei der Auswahl im Block 23 können eines oder mehrere der zuvor genannten Kriterien sein.

Dementsprechend erfolgt im Block 24 eine in zweifacher Hinsicht an Alter und Entwicklungsstand des Patienten angepasste Stadieneinteilung des Absens-EEG. Durch die Auswahl der geeigneten Klassifikationsfunktionen in Block 23 wird die Stadieneinteilung hinsichtlich der weiteren Auswertung der EEG-Kurven optimiert. Die hieraus gewonnenen Daten werden dabei nicht, wie bei bekannten Auswertegeräten, immer mit ein und demselben Schema der Stadieneinteilung klassifiziert, sondern variabel anhand des im Block 22 ausgewählten und für den jeweiligen Alters- bzw. Entwicklungsstand des Patienten optimierten Schemas der Stadieneinteilung klassifiziert. So wird z. B. bei einem erwachsenen Patienten für die Klassifikationsfunktion ein "Erwachsenenalgorithmus" ausgewählt. Die hierbei ermittelten Ergebnisse werden dann aufgrund des ausgewählten Schemas der Stadieneinteilung in eines von sechs Stadien A bis F einklassifiziert. Bei einem sehr jungen Kind würde als Klassifikationsfunktion ein "Kinderalgorithmus" verwendet werden. Für die Stadieneinteilung würde ein weniger feines Schema mit z. B. nur drei unterscheidbaren Stadien verwendet werden.

## Patentansprüche

1. Verfahren zur automatischen Auswertung eines Absens-EEG, bei dem EEG-Kurven von einem Auswertegerät (1) aufgenommen und mittels eines Rechners (2) des Auswertegerätes (1) ausgewertet werden, wobei bei der Auswertung aus den EEG-Kurven mittels des Rechners (2) anhand einer Stadieneinteilung des Absens-EEG wenigstens das aktuelle Stadium des Absens-Zustands eines Patienten bestimmt wird, wobei in der Stadieneinteilung Stadien der Tiefe des Absens-Zustands unterschieden werden, und wobei das aktuelle Stadium ausgegeben wird, **dadurch gekennzeichnet, dass** vom Rechner (2) ein bestimmtes Schema der Stadieneinteilung aus mehreren auswählbaren Schemata (26) von Stadieneinteilungen, die sich durch die Anzahl der unterscheidbaren Stadien des Absens-Zustands unterscheiden, ausgewählt wird und für die automatische Durchführung der Stadieneinteilung zur Bestimmung des aktuellen Stadiums verwendet wird.

2. Verfahren nach Anspruch 1, **dadurch gekennzeichnet, dass** eine Angabe über das Alter des Patienten, dessen EEG-Kurven aufgenommen werden, in das Auswertegerät (1) eingegeben wird und die automatische Auswahl des Schemas der Stadieneinteilung unter Berücksichtigung der eingegebenen Altersangabe vom Rechner (2) durchgeführt wird.

3. Verfahren nach einem der vorhergehenden Ansprüche, **dadurch gekennzeichnet, dass** die automatische Auswahl des Schemas der Stadieneinteilung unter Berücksichtigung der aufgenommenen EEG-Kurven und/oder daraus abgeleiteter Daten vom Rechner (2) durchgeführt wird.

4. Verfahren nach einem der vorhergehenden Ansprüche, **dadurch gekennzeichnet, dass** bei Erkennung eines bestimmten Mindestanteils niederfrequenter Signalanteile und Nichterkennung eines bestimmten Mindestanteils hochfrequenter Signalanteile in den EEG-Kurven ein Schema mit geringerer Anzahl der unterscheidbaren Stadien des Absens-Zustands ausgewählt wird als bei Erkennung eines bestimmten Mindestanteils hochfrequenter Signalanteile, die mit einem bestimmten Mindestanteil niederfrequenter Signalanteile kombiniert sein können.

5. Verfahren nach einem der vorhergehenden Ansprüche, **dadurch gekennzeichnet, dass** der Rechner (2) zunächst von einem ersten Schema mit einer bestimmten Anzahl der unterscheidbaren Stadien des Absens-Zustands ausgeht und zu einem Beurteilungszeitpunkt, zu dem eine ausreichende Anzahl von EEG-Daten vorliegt, bei Erkennung bestimmter Charakteristika in den EEG-Kurven ein zweites Schema der Stadieneinteilung auswählt, das eine geringere oder größere Anzahl der unterscheidbaren Stadien des Absens-Zustands aufweist als das erste Schema.

6. Verfahren nach einem der vorhergehenden Ansprüche, **dadurch gekennzeichnet, dass** wenigstens Frequenzanteile im Delta-Band als niederfrequente Signalanteile der EEG-Kurven gewertet werden.

7. Verfahren nach einem der vorhergehenden Ansprüche, **dadurch gekennzeichnet, dass** außer einem Standardschema der Stadieneinteilung ein weiteres Schema oder je nach Entwicklungsstufe des Patienten mehrere weitere Schemata der Stadieneinteilung vom Rechner (2) auswählbar sind, insbesondere Stadieneinteilungen mit einer geringeren Anzahl an unterscheidbaren Stadien des Absens-Zustands als das Standardschema.

8. Verfahren nach einem der vorhergehenden Ansprüche, **dadurch gekennzeichnet, dass** anhand des ausgewählten Schemas der Stadieneinteilung eine Angabe über den Entwicklungsstand des EEG bestimmt wird.

9. Verfahren nach einem der vorhergehenden Ansprüche, **dadurch gekennzeichnet, dass** die EEG-Kurven auf durch weitere Biosignale erzeugte Kurvenmuster analysiert werden und, wenn wenigstens ein solches Kurvenmuster erkannt wird, geprüft wird, ob ein anderes Schema für die Stadieneinteilung auszuwählen ist als bei Nichterkennung solcher Kurvenmuster.

10. Verfahren nach einem der vorhergehenden Ansprüche, **dadurch gekennzeichnet, dass** mittels Artefaktsensoren (5) Bewegungsartefakte in den aufgenommenen EEG-Kurven ermittelt werden und anhand der ermittelten Bewegungsartefakte die EEG-Kurven korrigiert und/oder die Stadieneinteilung korrigiert oder unterdrückt wird und/oder ein anderes Schema der Stadieneinteilung ausgewählt wird.

11. Computerprogramm mit Programmcodemitteln, insbesondere auf einem maschinenlesbaren Träger gespeichertes Computerprogramm, eingerichtet zur Durchführung eines Verfahrens nach einem der vorhergehenden Ansprüche, wenn das Computerprogramm auf einem Rechner (2) ausgeführt wird.

12. Auswertegerät (1) zur Auswertung eines Absens-EEG, wobei das Auswertegerät (1) wenigstens einen Rechner (2), EEG-Signalerfassungsmittel (7) und Ausgabemittel (3) aufweist, **dadurch gekennzeichnet, dass** das Auswertegerät (1) zur Durchführung eines Verfahrens nach einem der Ansprüche 1 bis 10 eingerichtet ist.

**Claims**

1. A method for automatically evaluating an absence EEG, in which EEG curves are recorded by an evaluation device (1) and evaluated by means of a computer (2) of the evaluation device (1), wherein at least the current stage of the absence state of a patient is determined during the evaluation from the EEG curves by means of the computer (2) on the basis of a stage division of the absence EEG, wherein a distinction is made in the stage division between stages of the depth of the absence state, and wherein the current stage is output, **characterized in that** a specific scheme of the stage division is selected by the computer (2) from a plurality of selectable schemes (26) of stage divisions, which differ in terms of the number of distinguishable stages of the absence state, and it is used for automatically carrying out the stage division for determining the current stage.

2. The method as claimed in claim 1, **characterized in that** a specification about the age of the patient, whose EEG curves are recorded, is entered into the evaluation device (1) and the automatic selection of the scheme of the stage division is carried out by the computer (2) taking into account the entered age specification.

3. The method as claimed in one of the preceding claims, **characterized in that** the automatic selection of the scheme of the stage division is carried out by the computer (2) taking into account the recorded EEG curves and/or data derived therefrom.

4. The method as claimed in one of the preceding claims, **characterized in that** a scheme with a smaller number of distinguishable stages of the absence state is selected in the case where a specific minimum portion of low-frequency signal components is identified and a specific minimum portion of high-frequency signal components is not identified in the EEG curves than in the case where a specific minimum portion of high-frequency signal components is identified, which may be combined with a specific minimum portion of low-frequency signal components.

5. The method as claimed in one of the preceding claims, **characterized in that** the computer (2) initially assumes a first scheme with a specific number of distinguishable stages of the absence state and, at an assessment time at which a sufficient amount of EEG data is available, it selects a second scheme of the stage division, which has a smaller or greater number of distinguishable stages of the absence state than the first scheme, if specific characteristics are identified in the EEG curves.

6. The method as claimed in one of the preceding claims, **characterized in that** at least frequency components in the delta band are judged to be low-frequency signal components of the EEG curves.

7. The method as claimed in one of the preceding claims, **characterized in that**, in addition to a standard scheme of the stage division, a further scheme or, depending on the development phase of the patient, a plurality of further schemes of the stage division are selectable by the computer (2), in particular stage divisions with a smaller number of distinguishable stages of the absence state than the standard scheme.

8. The method as claimed in one of the preceding claims, **characterized in that** a specification about the development phase of the EEG is determined on the basis of the selected scheme of the stage division.

9. The method as claimed in one of the preceding claims, **characterized in that** the EEG curves are analyzed in respect of curve patterns generated by further biosignals and, if at least one such curve pattern is identified, a check is carried out as to whether a different scheme for the stage division than in the case of a non-identification of such curve patterns is to be selected.

10. The method as claimed in one of the preceding claims, **characterized in that** movement artifacts in the recorded EEG curves are established by means of artifact sensors (5) and the EEG curves are corrected and/or the stage division is corrected or suppressed and/or a different scheme of the stage division is selected on the basis of the established movement artifacts.

11. A computer program with program code means, in particular a computer program stored on a machine-readable medium, configured to carry out a method as claimed in one of the preceding claims if the computer program is executed on a computer (2).

12. An evaluation device (1) for evaluating an absence EEG, wherein the evaluation device (1) has at least one computer (2), EEG signal detection means (7) and output means (3), **characterized in that** the evaluation device (1) is configured to carry out a method as claimed in one of claims 1 to 10.

**Revendications**

1. Procédé d'évaluation automatique d'un EEG d'absence, avec lequel des courbes d'EEG sont enregistrées par un appareil d'interprétation (1) et interprétées au moyen d'un ordinateur (2) de l'appareil d'interprétation (1), au moins le stade actuel de l'état d'absence d'un patient étant déterminé lors de l'interprétation à partir des courbes d'EEG au moyen de l'ordinateur (2) à l'aide d'une division en stades de l'EEG d'absence, des stades de la profondeur de l'état d'absence étant différenciés dans la division en stades, et le stade actuel étant délivré, **caractérisé en ce qu'**un schéma donné de la division en stades est sélectionné par l'ordinateur (2) parmi plusieurs schémas (26) pouvant être sélectionnés, lesquels se différencient par le nombre de stades différents de l'état d'absence, et utilisé pour la réalisation automatique de la division en stades en vue de déterminer le stade actuel.

2. Procédé selon la revendication 1, **caractérisé en ce qu'**une indication relative à l'âge du patient, dont les courbes d'EEG sont enregistrées, est saisie dans l'appareil d'interprétation (1) et la sélection automatique du schéma de la division en stades est effectuée par l'ordinateur (2) en tenant compte de l'indication d'âge saisie.

3. Procédé selon l'une des revendications précédentes, **caractérisé en ce que** la sélection automatique du schéma de la division en stades est effectuée par l'ordinateur (2) en tenant compte des courbes d'EEG enregistrées et/ou des données qui en sont dérivées.

4. Procédé selon l'une des revendications précédentes, **caractérisé en ce qu'**en cas de reconnaissance d'une part minimale définie de composantes de signal à basse fréquence et de non-reconnaissance d'une part minimale définie de composantes de signal à haute fréquence dans les courbes d'EEG, le schéma qui est alors sélectionné présente un plus petit nombre de stades de l'état d'absence différents que dans le cas de la reconnaissance d'une part minimale définie de composantes de signal à haute fréquence qui peuvent être combinées avec une part minimale définie de composantes de signal à basse fréquence.

5. Procédé selon l'une des revendications précédentes, **caractérisé en ce que** l'ordinateur (2) adopte tout d'abord un premier schéma présentant un nombre défini des différents stades de l'état d'absence et, à un instant d'évaluation auquel il existe un nombre suffisant de données d'EEG, en cas de reconnaissance de certaines caractéristiques dans les courbes d'EEG, sélectionne un deuxième schéma de la division en stades, lequel présente un nombre inférieur ou supérieur de stades différentiables de l'état d'absence que le premier schéma.

6. Procédé selon l'une des revendications précédentes, **caractérisé en ce qu'**au moins les composantes de fréquence dans la bande Delta sont valorisées en tant que composantes de signal à basse fréquence des courbes d'EEG.

7. Procédé selon l'une des revendications précédentes, **caractérisé en ce qu'**en plus d'un schéma standard de la division en stades, un schéma supplémentaire ou, suivant le niveau de développement du patient, plusieurs schémas supplémentaires de la division en stades peuvent être sélectionnés par l'ordinateur (2), notamment des divisions en stades avec un nombre de stades différentiables de l'état d'absence plus petit que celui du schéma standard.

8. Procédé selon l'une des revendications précédentes, **caractérisé en ce qu'**une indication relative à l'état de développement de l'EEG est définie au moyen du schéma sélectionné de la division en stades.

9. Procédé selon l'une des revendications précédentes, **caractérisé en ce que** les courbes d'EEG sont analysées pour y déceler des modèles de courbe générés par d'autres biosignaux et, lorsqu'au moins un tel modèle de courbe est reconnu, un contrôle est effectué pour vérifier s'il faut sélectionner un autre schéma pour la division en stades que dans le cas de la non-reconnaissance d'un tel modèle de courbe.

10. Procédé selon l'une des revendications précédentes, **caractérisé en ce que** des artefacts de mouvement dans les courbes d'EEG enregistrées sont déterminés au moyen de capteurs d'artefacts (5) et, à l'aide des artefacts de mouvement déterminés, les courbes d'EEG sont corrigées et/ou la division en stades est corrigée ou inhibée et/ou un autre schéma de la division en stades est sélectionné.

11. Programme informatique comprenant des moyens de code de programme, notamment programme informatique enregistré sur un support lisible par machine, conçu pour mettre en oeuvre un procédé selon l'une des revendications précédentes lorsque le programme informatique est exécuté sur un ordinateur (2).

12. Appareil d'interprétation (1) destiné à interpréter un EEG d'absence, l'appareil d'interprétation (1) possédant au moins un ordinateur (2), des moyens d'acquisition de signaux d'EEG (7) et des moyens de sortie (3), **caractérisé en ce que** l'appareil d'interprétation (1) est conçu pour mettre en oeuvre un procédé selon l'une des revendications 1 à 10.

Fig. 1

EP 3 019 080 B1

Fig. 3

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9715013 A2
- WO 2010034305 A1
- WO 2010034270 A1